# EUROPEAN PATENT APPLICATION

(11) **EP 3 701 867 A1**
(43) Date of publication of application: **02.09.2020**
(21) Application number: 19020097.2
(22) Date of filing: 28.02.2019
(51) Int. Cl.: A61B 5/22, A61B 5/00

(54) **ANALYSIS AND MEASUREMENT OF MAXIMUM AND EXPOLSIVE STRENGTH OF LOWER EXTREMITY MUSCLES**

(71) Applicant: Motus Melior d.o.o., 10000 Zagreb (HR)
(72) Inventor: MARKOVIC, Goran, 10000 Zagreb (HR); SARABON, Nejc, 4000 Kranj (SI)
(74) Representative: Boskovic, Davor

(57) **Abstract**

An apparatus for measuring maximum and explosive muscle strength of lower extremities in various positions whereby depending on the position, the strength of individual muscle groups is measured, consists of a basic casing (1), a mechanism for securing fastening which consists of a fixator (2) and belt (3), a mechanism for the angular movement of braces and for fastening (4) upon which braces (5) move and are fastened in a desired position, and a pair of braces with a sensor and lining (6), which are fastened and move in the mechanism for the angular movement of the braces (4), whereby linear movement of the braces (5) and angular rotation of the braces (5) are possible in each mechanism for the angular movement of the braces (4) at any angle, preferably at an angle of 90°.

## Description

### DESCRIPTION OF THE INVENTION

### Field of the invention

The subject invention relates to an innovative apparatus and method for the measurement of maximum and explosive strength of lower extremity muscles. The apparatus is intended for use on subjects that are mainly athletes in order to obtain results on maximum and explosive strength of lower extremity muscles of participants and for further risk assessment concerning injury incidence in comparison to obtained measurement results from a database of previously obtained results. According to the International Patent Classification, the subject invention is classified in class A61B 5/22 - Measuring muscular strength or the force of a muscular blow.

### Technical problem

The problem faced by the inventor is that a suitable apparatus for measuring multiple lower extremity muscle groups does not exist that would obtain results on maximum and explosive strength of lower extremity muscles of participants and the basis on which precise conclusions of the physical fitness of an observed subject can be provided, i.e. of the potential possibility of injury incidence of the lower extremities. Namely, numerous systems for measuring the load and strength of muscles exist, however, such systems do not provide uniform results and it is generally not possible to perform measurements of several muscles groups, i.e. to perform a larger number of measurements of various muscle groups.

The stated technical problem is solved using the subject invention conceptualized as a basic casing of a mechanism for securing the fastening comprising a fixator and a mechanism belt for angular motion of braces with fastening by which the braces move, and a pair of braces with force sensors that are attached to and move within the mechanism for the angular motion of the braces. The subject invention enables several different settings that are used for measuring the strength of various muscle groups, and in the following text, the fundamental positions for measuring and methods of positioning a subject will be described.

### Prior art

The nearest document from the prior art is document PCT/AU2012/001041, which also conceptualizes measuring muscle strength with a sensor; however, in respect to the subject invention, with the stated document it is only possible to measure forces exerted by only one upper leg muscle group, and movement or rotation of the measuring sensors is not possible, which would enable the measurement of more muscle groups and the measurement of a larger number of parameters.

The novelty of the subject patent in respect to prior art is the use of sensor braces, which can be moved and rotated 90°, providing the possibility of measuring the strength of muscles for a larger number of body regions and for a larger number of leg muscle groups.

### Disclosure of the invention

The subject apparatus for measuring the maximum and explosive strength of lower extremity muscles consists of three basic subsystems:
1. basic casing,
2. movable carriers with sensors,
3. measurement software.

The basic casing is constructed to provide multipurpose use. Its core is a surface where actions are performed that includes a system of belts for restraining the subject. The casing also includes a mechanical cradle for fastening two moveable carriers with built-in sensors. The bottom of the casing provides an area for storing the carriers and electronic equipment during transport, which ensures protection of the system's sensitive parts.

The basic casing is constructed to conform to mainstream design trends. The frame of the basic box-shaped casing is rigid, which is essential when taking measurements, as strong forces are exerted during such actions. The casing is also constructed like a chest, which enables easy transport. Measuring instructions are also displayed on the basic casing. The assembly of the basic casing consists of composite materials, which results in a relatively light weight combined with sufficient rigidity. The basic parts making up the basic casing are:
- main foundational assembly,
- metal rods for the fixing belts,
- slat for fastening the moveable carriers with sensors,
- lid portion for transport,
- transport wheels and peripheral elements.

The moveable carriers with sensors are mobile and symmetrical. The main requirement concerning the carriers is the minimum distance between the braces, which is achieved with the optimal geometry of their rotation. The change of distance between the braces (in a vertical and horizontal position) is achieved using a standard T-shaped cross-sectional element and placement points arranged in a grid of 20 mm. A special mechanism enables release with simple pulling. The rear side has T-shaped profiles that can slide up and down in the main profile's slot. The main functions that have been integrated in the assembly are:
1. the basic function is the unit for angular movement of segments of the body and for restraint in a desired position,
2. the second function is the integration of the fastening portion with sensors, which enables further length adjustments,
3. the third function is the assembly itself, which provides sufficient rigidity when taking measurements, while simultaneously being relatively light and offering enough space for hardware components.

Braces are tightly fastened to the sensors, which consist of cells for measuring single-axis loads. This type of sensor enables measuring at a specific distance from the sensor. The key characteristic of the apparatus according to the subject patent is the possibility of vertical and horizontal measurement of forces, which is achieved by rotating the braces 90°. During the stated rotation, the geometrical ratios for the most part do not change. The angular element, which supports the opposite side of the sensor, enables rotation, as well as tight fastening by using a special fastening mechanism. Geometrical properties are not significantly changed, which leads to adequate reliability for this type of measurement.

The interior part of the construction provides a space for the electronic components necessary for signal transmission and communication between both carriers and between the joint output connection and hardware equipment. The stated electrical components enable a visual display of data and the analysis thereof.

The basic parts of the inner assembly comprise the casing, the mechanism for securing the fastening, sideways from the starting point, the mechanism for the angular movement of the brace and fastening in the selected position, the brace with a sensor and lining, hardware sensors and communication devices, interior elements and protective gear.

The electronics consist of a printed circuit board, a battery and an antenna. Power for the circuit board is provided by the installed battery, and the circuit board has an antenna for achieving a larger wireless range. The electronics consist of several electronic chips, with the most important being the CPU and Bluetooth chips. The CPU chip takes care of the entire processing of the measurement results carried out by the circuit board, and the Bluetooth chip is used for communicating with a computer, desirably a tablet computer.

A software application is used to acquire, process and manage measurements. An application wirelessly connects to the electronics in the measuring modules and extracts data from them. Two types of tests are supported: maximum voluntary contraction and long-lasting submaximal contraction fatigue. The application is designed for carrying out tests with minimal user interaction.

The process for measuring the maximum and explosive muscle strength of the lower extremities using the apparatus includes the following steps:
a) selection of a subject from a database or the input of a subject if not in database,
b) selection of the type of test,
c) beginning of measurement, which includes positioning the subject in a position appropriate for taking measurements,
d) execution of the test by the subject one or more times, desirably twice,
e) stopping the test,
f) automatic analysis of acquired data, whereby results are compared as being greater than, equal to or less than reference values, and display of the results using software,
g) the acquired and calculated data are saved in a database for later use or manipulation.

### Brief description of the drawings

The subject invention is demonstrated in the figures that show:

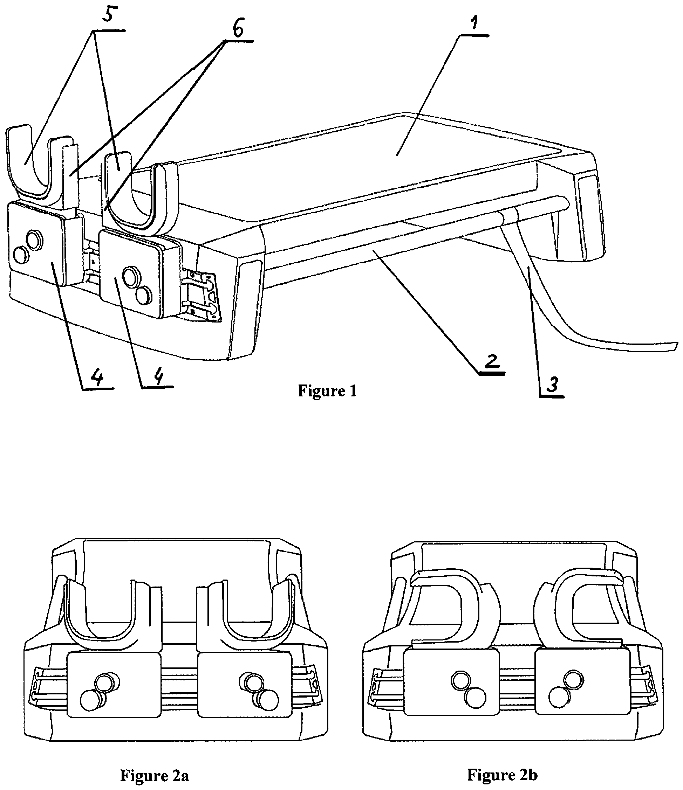

Figure 1: the basic assembly of the measuring board
Figures 2a and 2b: show the braces where they can be rotated 90°
Figure 3: cross section of the apparatus from figure 2a and the arrangement of internal components
Figure 4: shows the position of the subject for measurement of prone isometric hip extension
Figure 5: shows the position of the subject for measurement of supine isometric hip flexion
Figure 6: shows the position of the subject for measurement of supine isometric hip adduction
Figure 7: shows the position of the subject for measurement of supine isometric hip abduction
Figure 8: shows the position of the subject for measurement of kneeling isometric hip external rotation
Figure 9: shows the position of the subject for measurement of kneeling isometric hip internal rotation
Figure 10: shows the position of the subject for measurement of eccentric knee flexor strength

### Embodiment of the invention

The apparatus for measuring the maximum and explosive strength of lower extremity muscles comprises three basic subsystems: the basic casing (1), movable carriers with sensors (6) and measurement software. The basic casing (1), has a mechanism for securing the fastening comprising a fixator (2) and belt (3). The moveable braces (5) can be moved along a mechanism for angular movement of the braces (4) and for fastening, and are fastened in a selection position using a special fastening mechanism. The braces (5) contain a lining and a single-axis sensor (6), which measures the forces generated during measurements. The subject invention enables the linear movement of the braces (5), and the angular rotation of the braces (5) in each mechanism for the angular movement of the braces (4) at any angle, preferably at 90°.

In that manner, enabling linear and angular rotation of the braces allows for the measurement of maximum and explosive muscle strength of lower extremities for a larger number of different positions of a subject, which enables the measurement of several muscle groups as will be shown in the below-stated measurement examples.

### Examples of measurement positions and acquired experimental data

Thirty football players (19.1 ± 2.1 years of age; 1.79 ± 0.05 m; 74.4 ± 3.1 kg) participated in the research. None of the participants had injuries of the lower extremity muscles during the last 12 months. All test procedures were approved by a university's ethical committee for human research. Before testing, all participants or their parents (if <18 years old) gave written consent after all procedures were explained.

Participants visited the laboratory twice, with a 5-day break in between. Each testing procedure consisted of a 15-minute warm-up and measurement of isometric strength of seven different muscle groups by using the apparatus. The strength tests are the following: prone isometric hip extension, supine isometric hip flexion, supine isometric hip adduction, supine isometric hip abduction, kneeling isometric hip external rotation, kneeling isometric hip internal rotation, and eccentric knee flexor strength. Two rounds of testing were conducted for each strength test, whereby the pause between repetitions was 60 seconds, and 120 seconds between tests. The sequence of the testing was randomized.

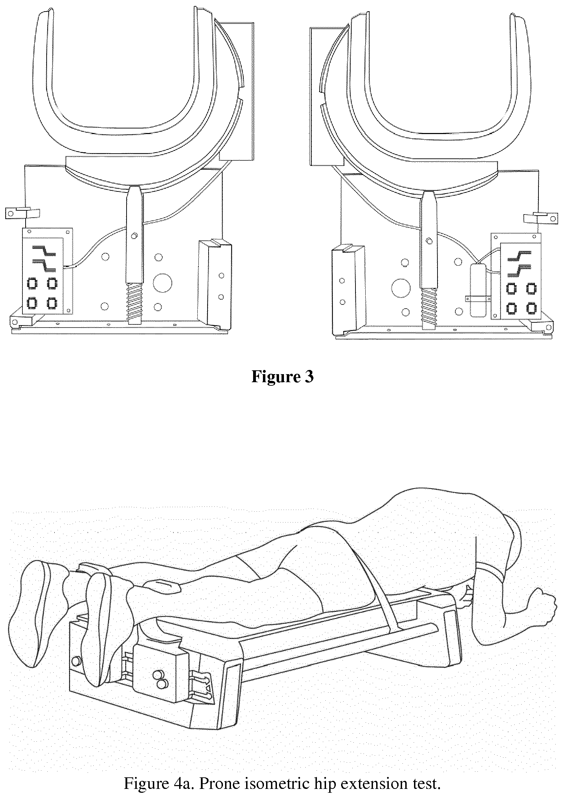

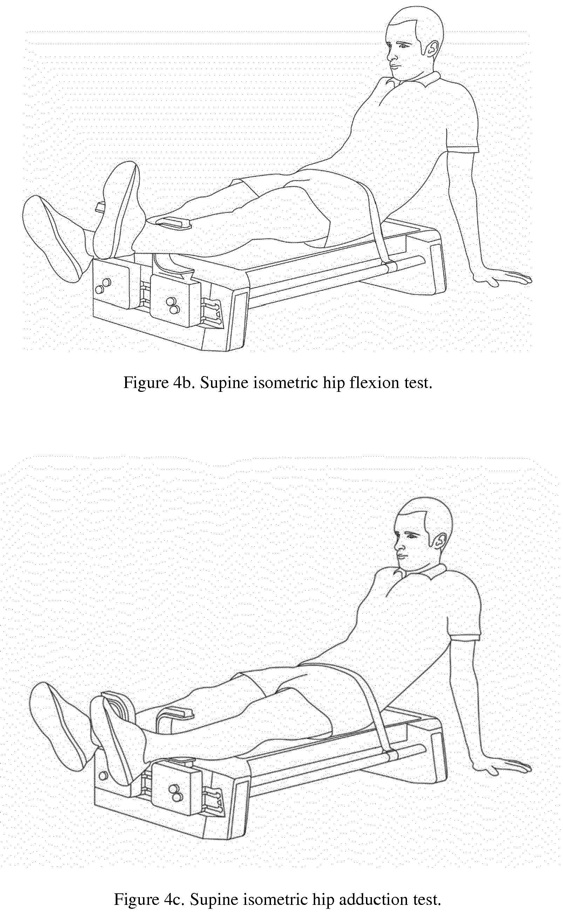

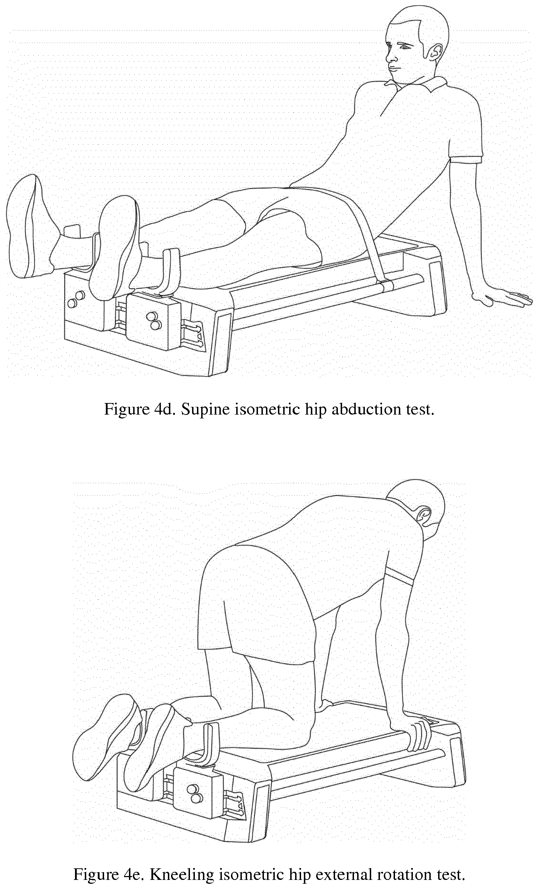

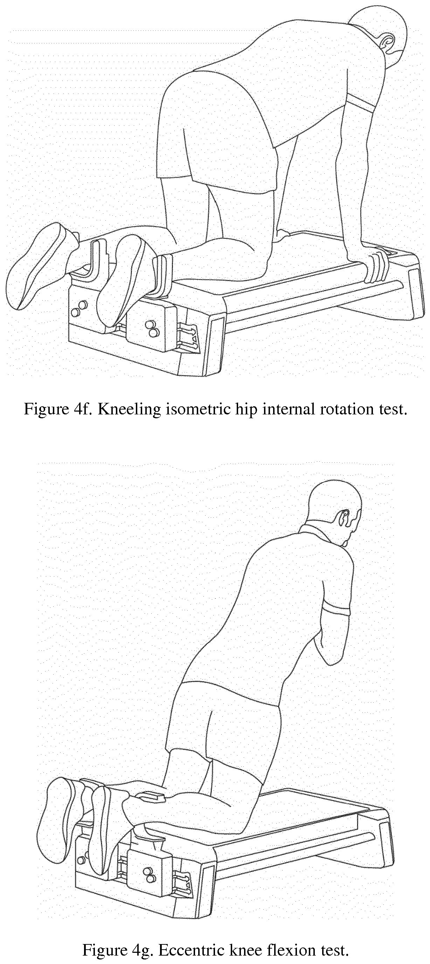

Prone isometric hip extension test (figure 4) was performed as follows. Participant was lying prone on the apparatus with one leg attached on the hook of the apparatus at the level of malleoli, and the other leg resting on the apparatus. A Velcro strap was used to stabilize the pelvis onto the apparatus. When ready, the participant performed maximal isometric hip extension with knee fully extended. Each leg was tested twice in randomized fashion.

Supine isometric hip flexion (figure 5) was performed as follows. Participant was lying supine on the apparatus with one leg attached on the hook of the apparatus at the level of malleoli, and the other leg resting on the apparatus. A Velcro strap was used to stabilize the pelvis onto the apparatus. When ready, the participant performed maximal isometric hip extension with knee fully extended. When ready, the participant performed maximal isometric hip extension with knee fully extended. Each leg was tested twice in randomized fashion.

Supine isometric hip adduction (figure 6) was performed as follows. Participant was sitting on the apparatus with both legs extended, attached on the hooks of the apparatus at the level of malleoli. When ready, the participant performed maximal bilateral isometric hip adduction with knees fully extended. Both legs were tested simultaneously twice.

Supine isometric hip abduction (figure 7) was performed as follows. Participant was sitting on the apparatus with both legs extended, attached on the hooks of the apparatus at the level of malleoli. When ready, the participant performed maximal bilateral isometric hip adduction with knees fully extended. Both legs were tested simultaneously twice.

Kneeling isometric hip external rotation (figure 8) was performed as follows. Participant was kneeling on the apparatus with hip and knee angles at 90 degrees, and with both legs attached on the hooks of the apparatus. When ready, the participant performed maximal isometric bilateral hip external rotation without moving knees or spine. Both legs were tested simultaneously twice.

Kneeling isometric hip internal rotation (figure 9) was performed as follows. Participant was kneeling on the apparatus with hip and knee angles at 90 degrees, and with both legs attached on the hooks of the apparatus. When ready, the participant performed maximal bilateral isometric hip internal rotation without moving knees or spine. Both legs were tested simultaneously twice.

Eccentric knee flexor test (figure 10) was performed as follows. Participant was kneeling on the apparatus, with the lower legs attached to the hooks of the apparatus at the level of malleoli. Arms were placed across the chest. When ready, participant slowly lowered the body towards the floor, while avoiding flexion of the hip. In case the participant could not lower his body under control, he was allowed to fall into the push-up position by placing the palms on the floor. Both legs were tested simultaneously twice.

Force data of all seven muscle strength tests were transferred to the computer at 1000 Hz through a 16-bit recording unit and stored for later analysis. The trial with the highest peak force in each test was used in further analyses. Descriptive statistics (means and standard deviations) for all force variables were calculated. Systematic bias was assessed using a t-test for dependent samples. Test-retest reliability for all tests was calculated using an intra-class correlation coefficient. All statistical analyses were done using a SPSS 15.0 software (SPSS Inc., Chicago, IL, USA). Level of statistical significance were set at p < 0.05. As there were no significant between-limb differences in maximum isometric force (all p > 0.05), only descriptive and reliability data for dominant (i.e. kicking) leg will be reported.

Descriptive statistics and test-retest correlation coefficients for all force variables for the dominant leg are presented in Table 1. No significant differences in maximum isometric force between the first and second measurement were observed for any of seven tests (all p > 0.05).

**Table 1. Means, standard deviations (SD) and intra-class correlation coefficients (ICC) for all seven strength tests measured on the apparatus.**

| Test | First measurement (mean ± SD) | Second measurement (mean ± SD) | ICC |
|---|---|---|---|
| Prone hip extension (N) | 283.7 ± 65.3 | 274.4 ± 63.4 | 0.85 |
| Supine hip flexion (N) | 180.2 ± 39.9 | 185.4 ± 39.7 | 0.91 |
| Supine hip abduction (N) | 213.6 ± 42.3 | 212.9 ± 41.1 | 0.88 |
| Supine hip adduction (N) | 238.4 ± 29.4 | 235.1 ± 31.2 | 0.93 |
| Kneeling hip external rotation (N) | 121.7 ± 25.6 | 126.0 ± 24.9 | 0.87 |
| Kneeling hip internal rotation (N) | 134.7 ± 27.5 | 137.2 ± 26.6 | 0.85 |
| Eccentric knee flexion (N) | 334.6 ± 46.5 | 318.9 ± 48.3 | 0.86 |

From the data presented above, the apparatus displays no significant systematic error and high level of test-retest reliability for all seven strength tests. It should be noted that the obtained test-retest reliability coefficients for selected strength tests are similar to those observed with expensive laboratory isokinetic hip or knee strength measurements (Almosnino et al. 2011; Julia et al. 2010; Maffiuletti et al. 2007). Almosnino S, Stevenson JM, Bardana DD, Diaconescu ED, Dvir Z. Reproducibility of isokinetic knee eccentric and concentric strength indices in asymptomatic young adults. Phys Ther Sport. 2012 Aug;13(3):156-62; Julia M, Dupeyron A, Laffont I, Parisaux JM, Lemoine F, Bousquet PJ, Hérisson C. Reproducibility of isokinetic peak torque assessments of the hip flexor and extensor muscles. Ann Phys Rehabil Med. 2010 Jun;53(5):293-305 and Maffiuletti NA, Bizzini M, Desbrosses K, Babault N, Munzinger U. Reliability of knee extension and flexion measurements using the Con-Trex isokinetic dynamometer. Clin Physiol Funct Imaging. 2007 Nov;27(6):346-53.

### Application of the invention

The subject application is intended for use for the measurement of maximum and explosive muscle strength of lower extremities in various positions whereby, depending on the position, the strength of individual muscle groups is measured. Persons skilled in the art will find it obvious that various changes and modifications to the described apparatus and method according to this invention can be made, without departing from the scope or essence of the invention.

## Claims

1. The apparatus for measuring maximum and explosive muscle strength of lower extremities comprising a basic casing (1), a mechanism for securing fastening consisting of a fixator (2) and a belt (3), a mechanism for angular movement of braces and for fastening (4) upon which braces (5) move and are fastened in a desired position, and a pair of braces with a sensor and lining (6), which are fastened and move in the mechanism for the angular movement of the braces (4)
**characterized in that** the linear movement of the braces (5) is possible, as well as angular rotation of the braces (5) in each mechanism for the angular movement of the braces (4) at any angle, preferably at an angle of 90°.

2. The method for measuring maximum and explosive muscle strength of lower extremities using the apparatus according to claim 1, is **characterized in that** the method involves the following steps:
a) selection of a subject from a database or the input of a subject if not in database,
b) selection of the type of test,
c) beginning of measurement, which includes positioning the subject in a position appropriate for taking measurements,
d) execution of the test by the subject one or more times, desirably twice,
e) stopping the test,
f) automatic analysis of acquired data, whereby results are compared as being greater than, equal to or less than reference values, and display of the results using software,
g) the acquired and calculated data are saved in a database for later use or manipulation.

3. The method for measuring maximum and explosive muscle strength of lower extremities using the apparatus according to claim 1, is **characterized in that** the method involves measuring the following measuring positions:
a) prone isometric hip extension, as shown in the Figure 4a
b) supine isometric hip flexion, as shown in the Figure 4b
c) supine isometric hip adduction, as shown in the Figure 4c
d) supine isometric hip abduction, as shown in the Figure 4d
e) kneeling isometric hip external rotation, as shown in the Figure 4e
f) kneeling isometric hip internal rotation, as shown in the Figure 4f te
g) eccentric knee flexor strength, as shown in the Figure 4g.
